# EUROPÄISCHE PATENTSCHRIFT

(11) **EP 2 713 970 B1**
(45) Veröffentlichungstag und Bekanntmachung des Hinweises auf die Patenterteilung: **22.07.2015**
(21) Anmeldenummer: 12723398.9
(22) Anmeldetag: 02.05.2012
(51) Int. Cl.: A61F 13/00, A61F 13/02, A61F 13/06

(54) **KOMPRESSIONSBINDE ZUM ANLEGEN AN DEN MENSCHLICHEN ODER TIERISCHEN KÖRPER**
COMPRESSION BANDAGE FOR PLACING ON THE HUMAN OR ANIMAL BODY
BANDAGE COMPRESSIF DESTINÉ À ÊTRE POSÉ SUR LE CORPS D'UN ÊTRE HUMAIN OU D'UN ANIMAL

(30) Priorität: 27.05.2011 DE 102011076596
(43) Veröffentlichungstag der Anmeldung: 09.04.2014
(73) Patentinhaber: Karl Otto Braun GmbH & Co. KG., 67752 Wolfstein (DE)
(72) Erfinder: JUNG, Harald, 67757 Kreimbach-Kaulbach (DE); KLÖPPELS, Michael, 52064 Aachen (DE)
(74) Vertreter: DREISS Patentanwälte PartG mbB
(86) Internationale Anmeldenummer: PCT/EP2012/058023
(87) Internationale Veröffentlichungsnummer: WO 2012/163616

(56) Entgegenhaltungen:
- EP-A1- 0 490 793
- WO-A1-98/47452
- WO-A1-2007/009625
- FR-A1- 2 433 935

## Beschreibung

Die Erfindung betrifft eine Kompressionsbinde zum Anlegen an den menschlichen oder tierischen Körper umfassend ein flächiges Bandmaterial mit einer Längsrichtung und einer Querrichtung sowie zwei sich in Längsrichtung gegenüberliegenden Querkanten und zwei sich in Querrichtung gegenüberliegenden Längskanten, wobei das Bandmaterial aus einem Gewebe besteht mit einem Kett- und einem Schussfadensystem, wobei wenigstens eines der Fadensysteme elastische Fäden umfasst. Kompressionsbinden sind im Stand der Technik vielfach bekannt.

So werden gewebte oder gewirkte elastische Binden sowohl in klebender als auch in nichtklebender Form verwendet, wobei ein Einsatz insbesondere in der Behandlung verschiedener venöser Zustände gegeben ist. Herkömmliche elastische gewobene oder gewirkte Binden besitzen ein Kettfaden- und ein Schussfadensystem, wobei insbesondere die Kettfäden elastisch ausgebildet sind. Dabei ist es wichtig, dass die Binden mit der richtigen Spannung angebracht werden, um eine gewünschte Kompressionskraft unter der Binde über einen vorgegebenen Zeitraum aufrechtzuerhalten. So werden in der Kompressionstherapie Binden eingesetzt, die über ihre gesamte Länge eine vorgegebene Dehnungsfähigkeit aufweisen und beispielsweise an einem Bein von distal nach proximal über die Ferse, den Knöchelbereich, den Unterschenkel und eventuell auch das Knie und den Oberschenkel angelegt werden. Probleme ergeben sich hierbei aus den unterschiedlichen Geometrien der umwickelten Bereiche, den Unterschieden im Anlegeverhalten und in der Kompressionswirkung. Dabei ist es erforderlich, dass entsprechende Binden einen Mindestkompressionsdruck über die gesamte Binde gewährleisten.

Bei herkömmlichen Ausgestaltungen einer solchen Kompressionsbinde kann es zu Schwierigkeiten beim Anlegen beispielsweise im Fersenbereich kommen und die unterschiedlichen Geometrien der zu umwickelnden Bereiche können nicht hinreichend berücksichtigt werden. Dadurch kommt es zu unterschiedlichen Kompressionswirkungen, die punktuell oder abschnittsweise nicht veränderbar bzw. beeinflussbar sind. Daher werden bisher, um auf die Unterschiede der zu umwickelnden Geometrien einzugehen, Binden mit unterschiedlichen Breiten angeboten. Dies sind sowohl Einzelbinden als auch Kombipacks mit Binden unterschiedlicher Breite, die nacheinander bzw. übereinander angewickelt werden.

Darüber hinaus ist beispielsweise aus der WO 98/47452 eine Kompressionsbandage zum Anlegen an einen Körperteil aus einem gewobenen oder gewirkten elastischen Bandagenmaterial vorbekannt, wobei eine Führungslinie zum Anlegen vorgesehen ist, die zu zumindest einer Kante der Bandage nicht parallel verläuft. Insbesondere soll die Bandage trapezförmig ausgebildet sein von einem in Längsrichtung angeordneten Ende zu einem weiteren in Längsrichtung davon beabstandeten Ende, wobei das schmalere Ende so an eine Gliedmaße angelegt wird, dass es dort zu liegen kommt, wo die höhere Kompressionskraft benötigt wird, z. B. im Bereich des Knöchels, und der breitere Bereich der Bandage beispielsweise im Wadenbereich appliziert wird, wo eine geringere Kompressionskraft vorgesehen sein soll.

Es ist nun Aufgabe der vorliegenden Erfindung, eine Kompressionsbinde bereitzustellen, die beim Anlegen die unterschiedlichen Dimensionen und Geometrien einer Gliedmaße und die unterschiedliche Kompressionskraft, die an die verschiedenen Bereiche einer Gliedmaße angelegt werden sollte, berücksichtigt.

Die Erfindung löst diese Aufgabe durch eine Kompressionsbinde, bei der die Dichte der Fäden im Kett- und/oder Schussfadensystem in Längsrichtung der Bandage variiert ist, so dass wenigstens ein Abschnitt der Bandage in Längsrichtung besteht, der eine andere Kett- und/oder Schussfadendichte aufweist, als ein benachbarter Abschnitt.

Durch die vorliegende Gestaltung können gegenüber einer Binde, wie sie normalerweise in der Kompressionstherapie eingesetzt wird, die über ihre gesamte Länge dieselben Eigenschaften aufweist, die sich aus unterschiedlichen Geometrien der umwickelten Bereiche, dem Anlegeverhalten und der Kompressionswirkung ergebenden Unterschiede flexibel berücksichtigt werden. Z. B. können Binden erzeugt werden, die in ihrer Längsrichtung an die unterschiedlichen Beinbereiche angepasste Eigenschaften aufweisen, durch die Vorsehung verschiedener Dichten von Kett- bzw. Schussfäden in mindestens zwei in Längsrichtungen aufeinanderfolgenden Bereichen. Auf diese Weise kann das Anlegeverhalten und die Wirkung der Kompressionsbinde verbessert werden.

Dabei kann über die Variation der Kettfaden- und/oder Schussfadendichte das Flächengewicht der Binde oder Bandage sowie die Dehnungsfähigkeit und Kompressionskraft eingestellt werden, wobei vorgesehen sein kann, dass eine Mindestkompressionskraft über die gesamte Binde gewährleistet bleibt.

Insbesondere sind die Eigenschaften der Binde hinsichtlich der drei vorgenannten Parameter über die Länge der Binde variabel einstellbar und individuell anpassbar. Hierdurch kann ein verbessertes Anlegeverhalten erreicht werden und der Kompressionsdruck über die Länge einer zu umwickelnden Extremität partiell eingestellt werden. Es ist dabei denkbar, auf Basis von Vermessungen der Extremität Binden individuell für jeden Patienten anzupassen.

Dabei ist vorgesehen, dass mit der Variation der Anzahl der Kett- und/oder Schussfäden in einem Kettfaden- oder Schussfadensystem die Kettfadendichte oder die Schussfadendichte, also die Anzahl der Fäden pro Zentimeter Bandagenbreite bzw. Bandagenlänge, variiert werden.

Es kann weiterhin vorgesehen sein, dass die Kettfäden elastisch ausgebildet sind, die Schussfäden jedoch unelastisch ausgestaltet sind. Alternativ können sowohl Kett- als auch Schussfäden elastisch sein, so dass eine sowohl in Längs- als auch in Querrichtung bielastische Binde geschaffen wird. Besonders bevorzugt ist es dabei, dass die elastischen Fäden baumwollelastische Fäden sind, wobei insbesondere Baumwollkreppfäden eingesetzt werden können.

Die Dehnbarkeit der Bandage bzw. Binde lässt sich über die Schussdichte bzw. über die Dicke der Schussgarne in gewissen Bereichen steuern und berechnen.

Durch überdrehte Baumwollgarne bzw. -zwirne ist es möglich, die gewünschte Dehnbarkeit und Kompressionskraft einzustellen. Die überdrehten Zwirne oder Garne, vorzugsweise aus Baumwolle, geben ihre überschüssige Energie im Gewebe ab, indem sie sich durch eine Behandlung mit Wasser und Tensiden verkürzen und dadurch elastifiziert werden.

Baumwollelastische Kreppfäden beeinflussen die Wirksamkeit von Kompressionsbinden deutlich. Insbesondere baumwollelastische Kreppfäden liefern einen Fertigverband mit geringem Ruhedruck bei gewünschtem Arbeitsdruck aufgrund ihres textilen Aufbaus und ihrer textilen Konstruktion.

Darüber hinaus können auch permanentelastische oder dauerelastische Materialien in Kombination mit anderen Garn- und/oder Faserarten zum Einsatz kommen. So können beispielsweise texturierte, thermoplastische Materialien eingesetzt werden, wie sie im Stand der Technik bereits in dauerelastischen Langzugbinden bzw. in permanentelastischen Binden eingesetzt werden, z. B. in der Binde "Lastodur straff" der Paul Hartmann AG, Heidenheim, Deutschland.

Unter Kettfäden sind dabei solche Fäden zu verstehen, die in Längsrichtung der Binde verlaufen, wobei Schussfäden, die in Querrichtung hierzu verlaufenden Fäden sind.

Dabei sollen definitionsgemäß die Begriffe "Binde" und "Bandage" synonym verwendet werden.

Besonders bevorzugt ist es dabei, wenn die Kettfadendichte zweier in Längsrichtung benachbarter Abschnitte bei gleicher Schussfadendichte unterschiedlich ist. Alternativ kann auch die umgekehrte Ausgestaltung vorgesehen sein, d. h. die Schussfadendichte zweier in Längsrichtung beabstandeter Abschnitte ist bei gleicher Kettfadendichte unterschiedlich. Durch die zusätzliche Variation der Schussfadendichte ist es möglich, die Dehnung zu steuern, um den Kompressionsdruck weiter wunschgemäß einzustellen. Generell können jedoch auch die Kett- und die Schussfadendichte beide variiert werden, insbesondere in verschiedenen und/oder gleichen Abschnitten der Binde.

Durch die Verdichtung im Kettfadenbereich bei einer längselastischen Binde wird eine Erhöhung der Kompressionskraft erzielt. Dies ist z. B. wünschenswert im Bereich des Knöchels und im unteren Bereich der Wade. Da die Verdichtung nicht sprunghaft erfolgt, wird eine Kompressionskraftreduktion von distal nach proximal allein schon durch die in der Kettfadendichte veränderte Webart erzielt. Bei gleichzeitiger Reduktion der Schussdichte kann die Dehnbarkeit erhöht und bei Erhöhung der Schussdichte die Dehnbarkeit reduziert werden.

Besonders bevorzugt kann weiterhin sein, dass die Abschnitte verschiedener Fadendichte eine unterschiedliche Erstreckung in Breitenrichtung aufweisen. Dabei können z. B. insbesondere die Bereiche mit geringerer Kettfadendichte eine größere Breite aufweisen als die Bereiche mit größerer Kettfadendichte. Auf diese Weise kann die Dichte bei gleicher Fadenanzahl z. B. des Kettfadens verändert werden.

Darüber hinaus kann die Kompressionsbinde weitere Lagen aufweisen, insbesondere können klebende Lagen, die insbesondere in Form einer klebenden Beschichtung auf dem Gewebe des Bandmaterials aufgebracht sind, vorgesehen sein. Die klebende Beschichtung kann dabei sowohl adhäsiver als auch rein kohäsiver Natur sein, d. h. lediglich so ausgestaltet sein, dass sie auf sich selber, nicht an Haut und Haaren sowie an der Bekleidung eines Trägers haftet.

Insbesondere ist vorgesehen, dass mehr als zwei Abschnitte unterschiedlicher Kettfaden- bzw. Schussfadendichte vorgesehen sind. Insbesondere kann vorgesehen sein, dass zwei Bereiche mit geringerer Kettfadendichte und/oder Schussfadendichte einen Bereich mit höherer Kettfadendichte und/oder Schussfadendichte zwischen sich in Längsrichtung einschließen. Sofern dabei vorgesehen ist, dass gleichzeitig mit der Variation der Fadendichte auch eine Variation der Erstreckung der Binde in Breitenrichtung vorgesehen ist, kann diese so ausgestaltet sein, dass innerhalb der Bereiche konstanter Fadendichte die Längskanten, die einander in Querrichtung gegenüberliegen, im Wesentlichen parallel ausgestaltet sind und lediglich im Übergangsbereich von einer Fadendichte zur nächsten Fadendichte es zu einer Verjüngung oder Erweiterung kommt, so dass ein trapezförmiger Abschnitt der Kompressionsbinde entsteht.

Eine besonders bevorzugte Ausführungsform liegt in einer Kurzzugbinde mit alternierender Breite und Kompressionsdruck, wobei hier ein Bereich geringerer Kettfadendichte und/oder Schussfadendichte jeweils im Bereich der in Längsrichtung vorgesehenen Endbereiche der Binde vorgesehen sind und dazwischen ein Bereich mit höherer Kettfadendichte und/oder Schussfadendichte. Die Bereiche mit verschiedener Kettfadendichte können dabei deckungsgleich zu den Bereichen mit verschiedener Schussfadendichte sein, können sich jedoch auch nicht oder nur teilweise überlappen.

Weiterhin kann die Kettfadendichte des Bereichs mit geringerer Kettfadendichte zu der Kettfadendichte des Bereichs mit größerer Kettfadendichte ein Verhältnis von 4:5, insbesondere von 170 bis 220 Fäden pro 10 cm und insbesondere von 172 bis 192 Fäden pro 10 cm aufweisen.

Die Schussfadendichte des Bereichs mit geringerer Schussfadendichte kann zu der Schussfadendichte des Bereichs mit der größeren Schussfadendichte ein Verhältnis von 0,8 bis 1,2, insbesondere von 130 bis 180 Fäden pro 10 cm und insbesondere von 140 bis 150 Fäden pro 10 cm aufweisen.

Schließlich kann bevorzugt vorgesehen sein, dass die Kompressionsbinde auf mindestens einer Seite des flächigen Bandmaterials eine Beschichtung, insbesondere eine kohäsive oder adhäsive Beschichtung, aufweist.

Eine entsprechende Kurzzugbandage kann dabei wie folgt ausgestaltet sein:

### Beispiel 1: variierende Kettfadendichte

Teil 1 und Teil 3 der Bandage in Längsrichtung
Materialien: Baumwollkreppfaden, 25 tex x 2 T/N ca. 2000 S + Z
Kettfadenanzahl: 86 fdn. - S + 86 fdn. - Z
Kettrapport: 2 S - 2 Z
Kettdichte in cm: 17,2
Bindenbreite: 10 cm
Kompressionsdruck bei 50 %iger Dehnung und zweilagiger Wicklung
Beindurchmesser: 12 cm 27 mm hg

Teil 2 (zwischen Teilen 1 und 3) der Bandage in Längsrichtung
Materialien: Baumwollkreppfaden, 25 tex x 2 T/N ca. 2000 S + Z
Kettfadenanzahl: 86 fdn. - S + 86 fdn. - Z
Kettrapport: 2 S - 2 Z
Kettdichte in cm: 21,5
Bindenbreite: 8 cm

Kompressionsdruck bei 50 %iger Dehnung und zweilagiger Wicklung
Beindurchmesser: 12 cm
Kompressionsdruck: 34 mm hg
Länge der Binde in Teil 1 und Teil 3: ca. 3 m gedehnt
Länge der Binde in Teil 2: ca. 2 m gedehnt.

### Beispiel 2: variierende Schussfadendichte in Längsrichtung der Binde

Für alle Abschnitte in Längsrichtung:
Bindenbreite: 10 cm
Material: Baumwollfaden der Stärke 36 tex, einfach
Schussfadendichte in Abschnitt 1 und 3 (vor und nach Schussfadendichte-Veränderung): 146 Fäden pro 10 cm
Kompressionsdruck bei 50%iger Dehnung und zweilagiger Wicklung und einem Beindurchmesser von 12 cm: 27 mm Hg
Schussfadendichte in Abschnitt 2 (während Schussfadendichte-Veränderung und zwischen den Abschnitten 1 und 3): 180 Fäden pro 10 cm
Kompressionsdruck bei 50%iger Dehnung und zweilagiger Wicklung und einem Beindurchmesser von 12 cm: 33 mm Hg

### Beispiel 3: Variation von Kett- und Schussfadendichte in einem Abschnitt.

Für alle Längenabschnitte:
Material Kettfaden: Baumwollkreppfaden, 25 tex x 2 T/N ca. 2000 S + Z
Material Schussfaden: Baumwollfaden 36 tex x 1
Kettrapport: 86 Fäden S + 86 Fäden Z
Längenabschnitt Teil 1 und Teil 3 (vor und nach Kett- und Schussfadendichte-Veränderung, in Längsrichtung der Binde): Bindenbreite 10 cm
Kettfadendichte: 172 Fäden pro 10 cm
Schussfadendichte: 146 Fäden pro 10 cm
Kompressionsdruck bei 50%iger Dehnung und zweilagiger Wicklung und einem Beindurchmesser von 12 cm: 27 mm Hg
Längenabschnitt Teil 2 (während Kett- und Schussfadendichte-Veränderung, zwischen Teilen 1 und 3): Bindenbreite 8 cm
Kettfadendichte 215 Fäden pro 10 cm
Schussfadendichte 180 Fäden pro 10 cm
Kompressionsdruck bei 50%iger Dehnung und zweilagiger Wicklung und einem Beindurchmesser von 12 cm: 41 mm Hg

Derartige Binden werden auf einer elektronisch gesteuerten Webmaschine mit differierenden Blattbreiten und Blattdichten gewebt. Die Ausrüstung und Konfektionierung erfolgt kontinuierlich an Endlosbändern. An den oben stehenden Angaben zu einer bevorzugten Ausführungsform einer derartigen Kompressionsbandage kann ersehen werden, wie durch die Einstellung der Kettfadenzahl pro cm durch die Änderung der Bindenbreite der Kompressionsdruck variiert werden kann. Der mit der Binde erzeugbare Kompressionsdruck kann als Ruhedruck in vivo am ruhenden, liegenden, menschlichen Bein mit einem Druckmessgerät der Firma Kikuhime gemessen werden. Dabei wird der Drucksensor des Druckmessgeräts zwischen angewinkelter Binde und der Haut am Übergang von der Achillessehne zum Soleusmuskel (entspricht dem in der Norm RAL GZ 387 festgelegten Messpunkt B1) platziert und es werden zwei Binden in zirkulärer Wicklung mit dabei nach Fertigstellung der Wicklung über dem Drucksensor liegenden vier Lagen übereinander angewickelt.

Die Erfindung wird im Folgenden anhand einer Zeichnung näher erläutert. Dabei zeigt:
Figur 1 einen Ausschnitt aus einer erfindungsgemäßen Kompressionsbinde;
Figur 2 eine stark schematisierte Darstellung zweier Bereiche einer erfindungsgemäßen Binde mit unterschiedlichen Kettfadendichten;
Figur 3 eine stark schematisierte Darstellung zweier Bereiche einer erfindungsgemäßen Binde mit unterschiedlicher Schussfadendichte und
Figur 4 eine stark schematisierte Darstellung zweier Bereiche mit unterschiedlichen Kett- und Schussfadendichten.

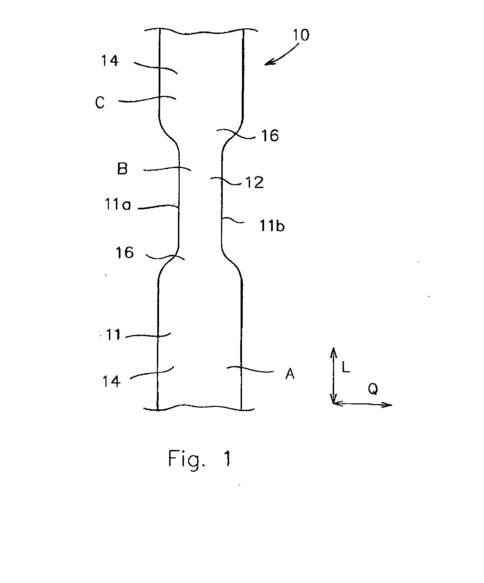

Figur 1 zeigt einen Ausschnitt einer erfindungsgemäßen Kompressionsbinde 10 umfassend ein flächiges Bandmaterial 11 mit einer Längsrichtung L und einer Querrichtung Q sowie zwei Längskanten 11a und 11b und zwei nicht dargestellten sich ebenfalls gegenüberliegenden Querkanten.

Die Kettfäden sind hier elastisch, insbesondere baumwollelastisch ausgebildet. Die Schussfäden sind unelastisch. Die Binde ist dabei im Wesentlichen in drei Abschnitte A, B, C gegliedert, wobei ein Abschnitt B mit höherer Kettfadendichte 12 durch zwei Abschnitte A, C mit geringerer Kettfadendichte 14 eingefasst ist. Die Abschnitte A, B und C schließen sich in Längsrichtung L aneinander an. Dabei sind im Bereich der Abschnitte A, B, C die Längskanten 11a, 11b, der Binde 10, die einander in Querrichtung Q gegenüberliegen, im Wesentlichen parallel zueinander angeordnet. Im Bereich des Übergangs von einem Abschnitt A, C mit geringer Kettfadendichte 14 zu einem Abschnitt B mit hoher Kettfadendichte 12 kommt es zu einer Verjüngung bzw. Verbreiterung der Binde 10, so dass hier trapezförmige Bereiche 16 entstehen.

Dabei ergibt sich durch Variation der Bindenbreite bei gleicher Kettfadenzahl eine Änderung in der Kettfadendichte.

Beim Anlegen an z. B. ein menschliches Bein kann im Bereich B der Binde 10 eine höhere Kompressionskraft erzielt werden, als in den Bereichen A und C. Darüber hinaus ermöglicht auch die verschiedene Bindenbreite neben der damit einhergehenden höheren Kompressionskraft ein leichtes Anlegen an stark vom Zylindrischen oder Konischen abweichende Bereiche eines menschlichen Körpers, wie z. B. dem Fuß- oder Knöchelbereich.

Auf die Verwendung verschieden breiter Binden 10, die miteinander kombiniert werden müssen, kann dann verzichtet werden.

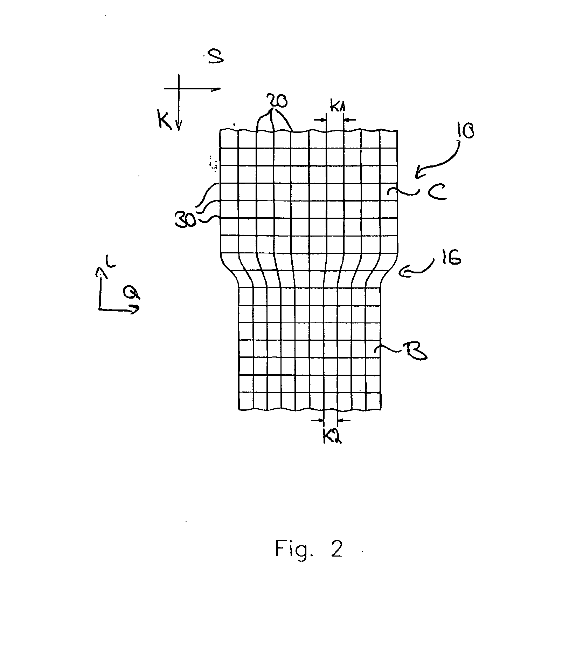

Figur 2 zeigt nun einen Ausschnitt aus der Binde 10 aus den Bereichen C und B sowie dem Übergangsbereich 16. Die Kettfäden sind mit dem Bezugszeichen 20 und die Schussfäden mit dem Bezugszeichen 30 versehen. Die Schussrichtung ist mit dem Buchstaben S und die Kettrichtung mit dem Buchstaben K gekennzeichnet. Dabei bleibt die Dichte der Schussfäden 30 über die gesamte Länge L der Binde konstant, wobei die Dichte der Kettfäden 20 vom Abschnitt C mit geringerer Kettfadendichte 14 zum Abschnitt B mit höherer Kettfadendichte erhöht ist, indem die Bindenbreite in Querrichtung Q im Abschnitt B geringer ist als im Abschnitt C. Hierdurch ergibt sich ein Übergangsbereich 16, der eine im Wesentlichen trapezförmige Form aufweist, sich insbesondere kontinuierlich ausgehend vom Bereich C zum Bereich B verjüngt. Hierbei besitzen im Bereich C die Kettfäden den Abstand K₁, wobei im Bereich B der Abstand der Kettfäden K₂ beträgt und K₂ kleiner K₁ ist.

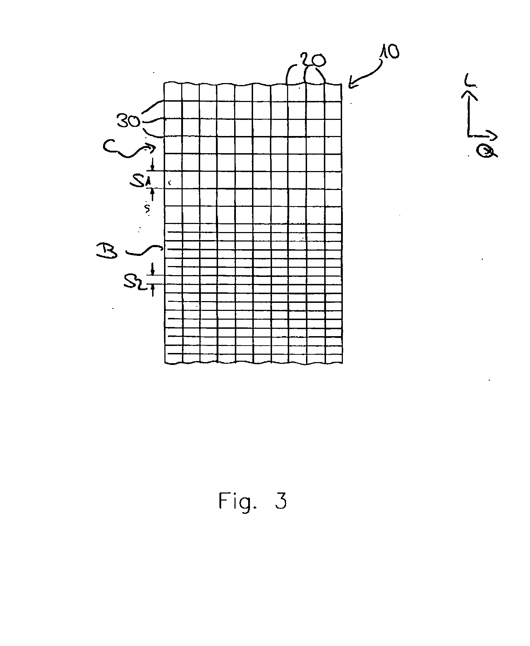

Figur 3 zeigt nun eine Gestaltung, bei der die Dichte und damit die Abstände der Kettfäden 20 über die gesamte Länge L der Binde 10 konstant bleibt und sich die Dichte der Schussfäden 30 über die Länge L ändert, wobei der Abstand der Schussfäden 30 im Bereich, der hier ebenfalls als C bezeichnet ist, mit S₁ bezeichnet ist und im Bereich, der ebenfalls als B bezeichnet ist und eine geringere Schussfadendichte aufweist, mit S₂. Dabei ist S₂ < S₁. Die Binde besitzt in diesem Fall über die gesamte Länge die gleiche Breite Q. Kann über die Variation der Kettfadendichte die Kompressionskraft eingestellt werden, so wird über die Variation der Schussfadendichte die Dehnbarkeit variiert. Darüber hinaus ändert sich durch die Variation der Schussfadendichte von einem Bereich mit geringerer Dichte C zu einem Bereich mit höherer Dichte B das Flächengewicht der Binde.

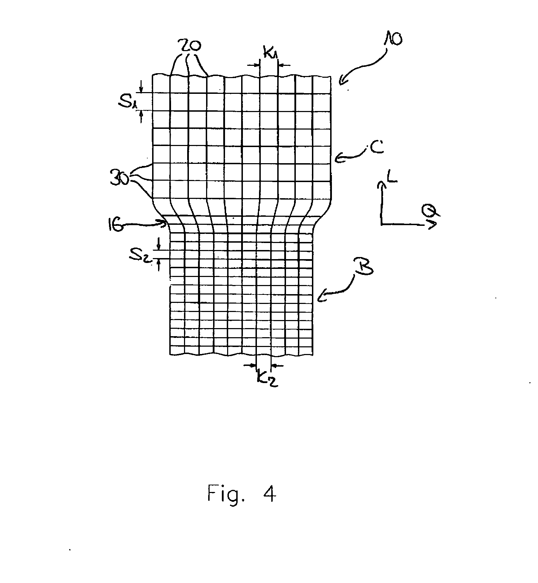

Schließlich zeigt Figur 4 eine Gestaltung, bei der sowohl ausgehend von einem Bereich B mit geringerer Schuss- und Kettfadendichte zu einem Bereich C, der eine höhere Kett- und Schussfadendichte aufweist, beide Fadensysteme hinsichtlich ihrer Dichte variiert werden. So weist im Bereich C die Binde 10 einen Abstand zwischen den Kettfäden von K₁ auf und einen Abstand zweier Schussfäden zueinander von S₁, wobei im Bereich B, der eine höhere Kett- und Schussfadendichte aufweist, die Schussfäden den Abstand S₂ aufweisen und die Kettfäden einen Abstand K₂. Dabei ist S₂ kleiner S₁ und K₂ kleiner K₁.

Auch hier wird die Variation der Kettfadendichte von einem Abstand K₁ auf einen Abstand K₂ durch eine Verschmälerung der Binde in Querrichtung Q erzielt, so dass sich ein Übergangsbereich 16 ausbildet, der wiederum eine sich verjüngende Form ausgehend von dem Bereich C zum Bereich B aufweist.

Durch die Variation sowohl der Kett- als auch der Schussfadensysteme hinsichtlich ihrer Dichte können sämtliche freien Parameter beliebig eingestellt werden und eine Binde geschaffen werden, die individuell und optimal an die Gegebenheiten, sofern gewünscht, auch an einen Träger anpassbar ist.

Weitere Ausführungsformen der Erfindung ergeben sich aus den übrigen Anmeldungsunterlagen.

## Patentansprüche

1. Kompressionsbinde zum Anlegen an den menschlichen oder tierischen Körper umfassend ein flächiges Bandmaterial (11) mit einer Längsrichtung (L) und einer Querrichtung (Q) sowie zwei sich in Längsrichtung (L) gegenüberliegenden Querkanten (11a, 11b) und zwei sich in Querrichtung (Q) gegenüberliegenden Längskanten, wobei das Bandmaterial (11) aus einem Gewebe besteht, mit einem Kett- (20) und einem Schussfadensystem (30), wobei wenigstens eines der Fadensysteme (20, 30) elastische Fäden umfasst, **dadurch gekennzeichnet, dass** die Fadendichte im Kett- und/oder Schussfadensystem (20, 30) in Längsrichtung (L) der Binde (10) variiert ist, so dass wenigstens ein Abschnitt (A, B, C) der Binde (10) in Längsrichtung (L) besteht, der eine andere Kett- und/oder Schussfadendichte aufweist, als ein benachbarter Abschnitt.

2. Kompressionsbinde nach Anspruch 1, **dadurch gekennzeichnet, dass** die Kettfäden (20) elastisch ausgebildet sind.

3. Kompressionsbinde nach Anspruch 1 oder 2, **dadurch gekennzeichnet, dass** die elastischen Fäden (20, 30) baumwollelastische Fäden sind.

4. Kompressionsbinde nach einem der vorangehenden Ansprüche, **dadurch gekennzeichnet, dass** die Kettfadendichte zweier in Längsrichtung (L) benachbarter Abschnitte (A, B, C) bei gleicher Schussfadendichte unterschiedlich ist.

5. Kompressionsbinde nach einem der vorangehenden Ansprüche 1 bis 3, **dadurch gekennzeichnet, dass** die Schussfadendichte zweier in Längsrichtung (L) benachbarter Abschnitte bei gleicher Kettfadendichte unterschiedlich ist.

6. Kompressionsbinde nach einem der vorangehenden Ansprüche 1 bis 3, **dadurch gekennzeichnet, dass** die Schussfadendichte sowie die Kettfadendichte zweier in Längsrichtung (L) benachbarter Abschnitte unterschiedlich ist.

7. Kompressionsbinde nach einem der vorangehenden Ansprüche, **dadurch gekennzeichnet, dass** die Abschnitte verschiedener Fadendichte eine unterschiedliche Erstreckung in Querrichtung (Q) aufweisen.

8. Kompressionsbinde nach einem der vorangehenden Ansprüche, **dadurch gekennzeichnet, dass** die Kettfadendichte des Bereichs (14) mit geringerer Kettfadendichte zu der Kettfadendichte des Bereichs mit größerer Kettfadendichte ein Verhältnis von 4:5, insbesondere von 170 bis 220 Fäden pro 10 cm und insbesondere von 172 bis 190 Fäden pro 10 cm aufweist.

9. Kompressionsbinde nach einem der vorangehenden Ansprüche, **dadurch gekennzeichnet, dass** die Schussfadendichte des Bereichs mit geringerer Schussfadendichte zu der Schussfadendichte des Bereichs mit der größeren Schussfadendichte ein Verhältnis von 0,8 bis 1,2, insbesondere von 130 bis 180 Fäden pro 10cm und insbesondere von 140 bis 150 Fäden pro 10 cm aufweist.

10. Kompressionsbinde nach einem der vorangehenden Ansprüche, **dadurch gekennzeichnet, dass** mindestens eine Seite des flächigen Bandmaterials eine Beschichtung, insbesondere eine kohäsive oder adhäsive Beschichtung, aufweist.

## Claims

1. Compression bandage for putting on the human or animal body including a planar band material (11) with a longitudinal direction (L) and a transverse direction (Q) and two transverse edges (11a, 11b) which oppose each other in longitudinal direction (L) and two longitudinal edges which oppose each other in transverse direction (Q) wherein the band material (11) consists of a fabric with a warp (20) and a weft thread system (30), wherein at least one of the thread systems (20, 30) includes elastic threads, **characterized in that** the thread density in the warp and/or weft thread system (20, 30) varies in longitudinal direction (L) of the bandage (10) so that at least one section (A, B, C) of the bandage (10) exists in longitudinal direction(L) which has a different warp and/or weft thread density than a neighboring section.

2. Compression bandage according to claim 1, **characterized in that** the warp threads (20) are configured elastic.

3. Compression bandage according to claim 1 or 2, **characterized in that** the elastic threads (20, 30) are cotton elastic threads.

4. Compression bandage according to one of the preceding claims **characterized in that** the warp thread density of two sections (A, B, C) that neighbor each other in longitudinal direction (L) are different at the same weft thread density.

5. Compression bandage according to one of the preceding claims 1 to 3, **characterized in that** the weft thread density of two sections which neighbor each other in longitudinal direction (L) is different at same warp thread density.

6. Compression bandage according to one of the preceding claims 1-3, **characterized in that**.the weft thread density and the warp thread density of two sections that neighbor each other in longitudinal direction (L) is different.

7. Compression bandage according to one of the preceding claims, **characterized in that** the sections of different thread density have a different extent in transverse direction (Q).

8. Compression bandage according to one of the preceding claims, **characterized in that** the warp thread density of the region (14) with lower warp thread density has a ration to the warp thread density of the region with greater warp thread density of 4:5, in particular of 170 to 220 threads per 10 cm and in particular from 172 to 190 threads per 10 cm.

9. Compression bandage according to one of the preceding claims, **characterized in that** the weft thread density of the region with lower weft thread density to the weft thread density of the region with the greater weft thread density has a ration of 0.8 to 1.2 in particular of 130 to 180 threads per 10 cm, and in particular from 140 to 150 threads per 10 cm.

10. Compression bandage according to one of the preceding claims, **characterized in that** at least one side of the planar band material has a coating in particular a cohesive or adhesive coating.

## Revendications

1. Bandage compressif destiné à être posé sur le corps d'un être humain ou d'un animal, comprenant un matériau plat en bande (11) présentant un sens longitudinal (L) et un sens transversal (Q) ainsi que deux bords transversaux (11a, 11b) opposés dans le sens longitudinal (L) et deux bords longitudinaux opposés dans le sens transversal (Q), le matériau en bande (11) étant constitué d'un tissu présentant un système de fils de chaîne (20) et de fils de trame (30), au moins un des systèmes de fils (20, 30) contenant des fils élastiques, **caractérisé en ce que** la densité des fils dans le système de fils de chaîne et/ou de trame (20, 30) varie dans le sens longitudinal (L) du bandage (10), de sorte qu'au moins une partie (A, B, C) du bandage (10) dans le sens longitudinal (L) présente une autre densité de fils de chaîne et/ou de trame qu'une partie voisine.

2. Bandage compressif selon la revendication 1, **caractérisé en ce que** les fils de chaîne (20) sont élastiques.

3. Bandage compressif selon la revendication 1 ou 2, **caractérisé en ce que** les fils élastiques (20, 30) sont des fils élastiques en coton.

4. Bandage compressif selon l'une quelconque des revendications précédentes, **caractérisé en ce que** la densité des fils de chaîne de deux parties (A, B, C) voisines dans le sens longitudinal (L), dans le cas d'une densité de fils de trame identique, est différente.

5. Bandage compressif selon l'une quelconque des revendications 1 à 3, **caractérisé en ce que** la densité des fils de trame de deux parties voisines dans le sens longitudinal (L), dans le cas d'une densité de fils de chaîne identique, est différente.

6. Bandage compressif selon l'une quelconque des revendications 1 à 3, **caractérisé en ce que** la densité des fils de trame ainsi que la densité des fils de chaîne de deux parties voisines dans le sens longitudinal (L) sont différentes.

7. Bandage compressif selon l'une quelconque des revendications précédentes, **caractérisé en ce que** les parties d'une densité de fils différente présentent une étendue différente dans le sens transversal (Q).

8. Bandage compressif selon l'une quelconque des revendications précédentes, **caractérisé en ce que** la densité des fils de chaîne de la zone (14) d'une densité de fils de chaîne très faible par rapport à la densité de fils de chaîne de la zone d'une densité de fils de chaîne très élevée présente un rapport de 4:5, en particulier de 170 à 220 fils pour 10 cm et en particulier de 172 à 190 fils pour 10 cm.

9. Bandage compressif selon l'une quelconque des revendications précédentes, **caractérisé en ce que** la densité de fils de frame de la zone d'une densité de fils de frame très faible par rapport à la densité de fils de trame de la zone d'une densité de fils de trame très élevée présente un rapport de 0,8 à 1,2, en particulier de 130 à 180 fils pour 10 cm et en particulier de 140 à 150 fils pour 10 cm.

10. Bandage compressif selon l'une quelconque des revendications précédentes, **caractérisé en ce qu'**au moins une face du matériau plat en bande présente un revêtement, en particulier un revêtement cohésif ou adhésif.
